# EUROPÄISCHE PATENTSCHRIFT

(11) **EP 3 512 761 B1**
(45) Veröffentlichungstag und Bekanntmachung des Hinweises auf die Patenterteilung: **08.07.2020**
(21) Anmeldenummer: 17765436.5
(22) Anmeldetag: 13.09.2017
(51) Int. Cl.: B62D 49/06, G01V 3/10

(54) **VORRICHTUNG ZUR ERFASSUNG DER BESCHAFFENHEIT EINES UNTERGRUNDS**
DEVICE FOR DETECTING THE PROPERTIES OF A SUBSURFACE
DISPOSITIF DE DÉTECTION DE LA NATURE D'UN SOL

(30) Priorität: 13.09.2016 AT 508162016
(43) Veröffentlichungstag der Anmeldung: 24.07.2019
(73) Patentinhaber: Geoprospectors GmbH, 2514 Traiskirchen (AT)
(72) Erfinder: PREGESBAUER, Michael, 2500 Baden (AT)
(74) Vertreter: Puchberger & Partner Patentanwälte
(86) Internationale Anmeldenummer: PCT/EP2017/072966
(87) Internationale Veröffentlichungsnummer: WO 2018/050665

(56) Entgegenhaltungen:
- EP-A2- 2 302 417
- WO-A1-2015/164791
- WO-A1-2017/092885
- US-A1- 2014 097 831

## Beschreibung

Die Erfindung betrifft eine Vorrichtung zur Erfassung der Beschaffenheit eines Untergrunds, eine landwirtschaftliche Arbeitsmaschine mit einer derartigen Vorrichtung sowie ein Verfahren zum Betrieb einer derartigen landwirtschaftlichen Arbeitsmaschine.

Aus dem Stand der Technik im Bereich der Geophysik ist es bekannt, Bodensensoren einzusetzen, welche auf Basis elektromagnetischer Induktion (EMI) die Beschaffenheit eines Untergrunds detektieren. Dabei wird von einer elektromagnetischen Sendespule ein Primärfeld erzeugt, welches im Untergrund in Abhängigkeit von der Bodenbeschaffenheit ein elektromagnetisches Sekundärfeld induziert, welches durch eine oder mehrere Empfangsspulen im Oberflächenbereich aufgenommen und ausgewertet wird.

Aus den Signalen der Empfangsspulen ergeben sich Leitfähigkeitswerte, aus denen, je nach der Konfiguration und Empfindlichkeit der Spulen, die Beschaffenheit des Untergrunds, beispielsweise die Dichte, die Wassersättigung und die Bodenart, in einer Tiefe von wenigen Zentimetern bis mehreren Metern bestimmt werden kann.

Da die EMI Technologie im Gegensatz zu anderen geophysikalischen Methoden bei nahezu allen Untergrundsituationen einsetzbar ist und auch vergleichsweise kostengünstig in der Anschaffung ist, hat sie sich als Messmethode in der teilflächenspezifischen Landwirtschaft (Precision Farming) etabliert. Der Hauptanwendungsbereich etablierter Systeme ist es, Bodeninhomogenitäten in eigenen Messfahrten lateral zu erfassen und danach dem Landwirt in Form von Karten und Plänen zur Verfügung zu stellen.

Dabei werden zum Teil motorisierte Systeme eingesetzt, die auf einem zumeist eigens konstruierten Anhängesystem montiert werden und über die Untersuchungsfläche gezogen werden. Der Bodensensor wird dabei hinter der Zugmaschine montiert und es muss ein großer Abstand des Bodensensors zum Zugfahrzeug eingehalten werden, um ein die Qualität minderndes Messrauschen oder sonstige Fehler bei der Datenerfassung möglichst zu verhindern.

Beim operativen Betrieb bekannter Bodensensoren findet die Datenerfassung unabhängig von Bewirtschaftungsfahrten des Landwirts statt, da der Betrieb des Systems und die Auswertung der Daten Fachpersonal erfordert. Von der Datenaufnahme bis zur Lieferung der Karten mit den ausgewerteten Bodendaten läuft somit ein komplexer Prozess ab, in den der Landwirt nicht eingebunden wird.

Aus der WO 2015/164791 A1 ist es bekannt, Bodenanalysatoren, welche auf verschiedenen Detektionsprinzipien basieren, am Vorderteil einer landwirtschaftlichen Arbeitsmaschine zu positionieren, um in einem Arbeitsgang eine Bodenanalyse und eine Bodenbearbeitung durchzuführen, wobei die Bearbeitung in Abhängigkeit von den gemessenen Daten erfolgen kann.

Aufgabe der Erfindung ist es, die Nachteile der bestehenden Systeme zu beheben und eine Vorrichtung zur Erfassung der Beschaffenheit eines Untergrunds sowie ein Verfahren zum Betrieb einer derartigen Vorrichtung zu realisieren, welche es dem Landwirt ermöglichen, flexibel und unabhängig bereits während der landwirtschaftlichen Arbeitsfahrt Bodeninformationen zu erfassen und diese auch gleichzeitig oder anschließend für eine Steuerung landwirtschaftlicher Arbeitsmaschinen zu verwenden.

Diese Aufgabe wird erfindungsgemäß durch eine Vorrichtung, eine landwirtschaftliche Arbeitsmaschine und ein Verfahren zum Betrieb einer landwirtschaftlichen Arbeitsmaschine gemäß den unabhängigen Patentansprüchen gelöst.

Die erfindungsgemäß vorgeschlagene Vorrichtung umfasst zumindest eine Sendespule und zumindest eine, vorzugsweise vier, Empfangsspulen, wobei die Sendespule zur Erzeugung eines elektromagnetischen Primärfelds und die Empfangsspule zum Empfangen des im Untergrund durch das Primärfeld induzierten elektromagnetischen Sekundärfelds eingerichtet ist. Sowohl die Sendespule als auch die Empfangsspule sind in einem Front- oder Ballastgewicht angeordnet. Derartige Front- oder Ballastgewichte werden bei landwirtschaftlichen Zugmaschinen an einer Aufhängung an der Vorderseite der Zugmaschine montiert und dienen einerseits zur Verbesserung der Traktion der Zugmaschine und andererseits, um ungleiche Belastung durch schwere am Heck der Zugmaschine montierte Geräte auszugleichen. Sie sind in der Regel aus Beton gegossen und weisen ein Gewicht von etwa einer Tonne auf.

Durch Anordnung der Sende- und Empfangsspule in einem Frontgewicht wird erreicht, dass die Erfassung der Beschaffenheit des Untergrunds bereits während einer Arbeitsfahrt der Zugmaschine erledigt werden kann. Da die Frontgewichte in der Regel aus Beton gegossen werden, wird auch maximale Schonung und Schutz der sensiblen elektronischen Bauteile erreicht. Die starre Verbindung zwischen der Zugmaschine und den Sensorbauteilen reduziert darüber hinaus bewegungsinduzierte Störungen.

Erfindungsgemäß ist vorgesehen, dass im Frontgewicht ein Sensoraufnahmebereich zur Aufnahme der Sende- und Empfangsspulen und ein davon räumlich getrennt angeordneter Elektronikaufnahmebereich zur Aufnahme der Steuerelektronik vorgesehen sind. Dies hat den Vorteil, dass die Sende- und Empfangsspulen an speziellen Bereichen des Frontgewichts, vorzugsweise im vorderen unteren Bereich, angeordnet werden können.

Das Frontgewicht kann erfindungsgemäß so dimensioniert werden, dass es neben der Sensorfunktionalität auch jenes Gewichtsmaß aufweist, um als Gegengewicht zu einem im Heck angeordneten Bodenbearbeitungsgerät zu fungieren.

Besonders vorteilhaft ist eine Ausführungsform der Erfindung, bei der der Sensoraufnahmebereich und Elektronikaufnahmebereich durch eine elektromagnetische Strahlen abschirmende Abschirmplatte getrennt sind. Bei dieser Abschirmplatte kann es sich vorzugsweise um eine Stahlplatte oder um eine Platte aus einem anderen elektromagnetische Strahlen abschirmenden Material handeln. Dadurch wird gewährleistet, dass die im Sensoraufnahmebereich angeordneten Sende- und Empfangsspulen nicht durch störende elektromagnetische Signale, insbesondere von der in unmittelbarer Nähe befindlichen Zugmaschine selbst, beeinflusst wird. Darüber hinaus wird durch die Abschirmplatte auch eine Abschirmung gegen mechanische Einflüsse erzielt. Erfindungsgemäß kann auch ein Anschluss vorgesehen sein, um die Abschirmplatte mit einer externen elektrischen Erdungsleitung zu verbinden. Erfindungsgemäß kann vorgesehen sein, dass das Frontgewicht einen Träger zur Montage des Frontgewichts an einer landwirtschaftlichen Arbeitsmaschine aufweist, wobei die Abschirmplatte zwischen dem Träger und dem Sensoraufnahmebereich angeordnet ist. Dadurch wird sichergestellt, dass sich bei korrekter Montage des Frontgewichts die Abschirmplatte zwischen der Arbeitsmaschine und den Sende- und Empfangsspulen befindet.

Erfindungsgemäß kann vorgesehen sein, dass zur Aufnahme der Sende- und Empfangsspulen im Sensoraufnahmebereich eine vorzugsweise U-förmige und nach unten offene Sensorabschirmung angeordnet ist. Die Sensorabschirmung kann insbesondere aus dem Sensoraufnahmebereich entnehmbar sein. Dies ermöglicht die Ausführung der erfindungsgemäßen Vorrichtung als modulares System: Die Sensorkomponenten können in das Frontgewicht eingebaut und aus diesem entfernt werden, sodass auch eine Nutzung der Sensorkomponenten ohne das Frontgewicht möglich ist.

Die Breite des Sensoraufnahmebereichs bzw. der Sensorabschirmung kann zur Aufnahme der Sensorkomponenten dem Maß für die maximale Sende- und Empfangsspulenkonfiguration entsprechen, also etwa 1681 mm aufweisen.

Die Sensorabschirmung kann ein Metall oder glasfaserverstärkten Kunststoff umfassen. Die Sensorabschirmung kann vorzugsweise mit einem Anschluss versehen sein, um sie mit einer externen elektrischen Erdungsleitung zu verbinden.

Erfindungsgemäß kann vorgesehen sein, dass ein vorzugsweise ultraschallbasierter Abstandssensor zur Bestimmung des Abstandes der Vorrichtung zum Untergrund vorgesehen ist. Der Abstandssensor kann vorzugsweise im unteren Bereich des Frontgewichts vorgesehen sein.

Erfindungsgemäß kann insbesondere vorgesehen sein, dass die Abschirmplatte zwischen dem Sensoraufnahmebereich und dem Abstandssensor angeordnet ist. Im Unterschied zu den Sende- und Empfangsspulen wird der Abstandssensor in der Regel durch elektromagnetische Störungen der Zugmaschine nicht beeinflusst, sodass eine Abschirmung nicht erforderlich ist.

Erfindungsgemäß kann vorgesehen sein, dass zumindest ein Neigungssensor zur Bestimmung der Neigung der Vorrichtung relativ zum Untergrund vorgesehen ist.

Erfindungsgemäß kann vorgesehen sein, dass zumindest ein Lokalisierungsmodul, vorzugsweise ein GPS-Modul, vorgesehen ist.

Das Frontgewicht kann vorzugsweise Beton umfassen oder zur Gänze aus Beton gegossen sein. Dabei kann die Abschirmplatte vorzugsweise eingegossen sein. Weiters kann vorgesehen sein, dass zur Gewährleistung der mechanischen Stabilität des Frontgewichts eine Stahlstangenkonstruktion eingegossen ist, die vorzugsweise derart im Frontgewicht angeordnet ist, dass sie über dem Sensoraufnahmebereich liegt. Dadurch wird gewährleistet, dass die Stahlstangen die Sensitivität der Sende- und Empfangsspulen nicht beeinflussen.

Weiters können Mittel zum Anschluss einer externen Recheneinheit und/oder eines externen Terminals vorgesehen sein. Der Sensoraufnahmebereich und der Elektronikaufnahmebereich können durch Kunststoffrohre miteinander verbunden sein, wobei die Kunststoffrohre neben oder unter der Abschirmplatte geführt sind.

Die Erfindung umfasst weiters eine landwirtschaftliche Arbeitsmaschine, insbesondere eine Zugmaschine, umfassend eine erfindungsgemäße Vorrichtung. Die erfindungsgemäße Vorrichtung kann vorzugsweise an einem Fronthubwerk der Arbeitsmaschine montiert sein.

Auf der Arbeitsmaschine kann eine Recheneinheit zur Umrechnung der empfangenen Signale in elektrische Leitfähigkeitswerte und Bodenparameter, beispielsweise Dichte, Feuchtigkeit und Bodenart, vorgesehen sein. Weiters können an der Arbeitsmaschine Steuerausgänge zur Ansteuerung externer Bodenbearbeitungsgeräte vorgesehen sein. Die Steuerausgänge können vorzugsweise im Heckbereich der Arbeitsmaschine angeordnet sein, sodass eine unmittelbare Ansteuerung von im Heckbereich der Arbeitsmaschine angeordneten Bodenbearbeitungsgeräten auf Grundlage der während einer Arbeitsfahrt detektierten Bodenbeschaffenheit ermöglicht wird.

Die Erfindung umfasst weiters ein Verfahren zum Betrieb einer erfindungsgemäßen landwirtschaftlichen Arbeitsmaschine, wobei während einer Arbeitsfahrt der Arbeitsmaschine die Bodenparameter des aktuell zu bearbeitenden Bodenabschnittes detektiert werden.

Die Vorrichtung kann vorzugsweise während der Arbeitsfahrten von der Arbeitsmaschine in einer Geschwindigkeit von maximal 15 km/h über den zu untersuchenden Untergrund bewegt werden, wobei die detektierte Beschaffenheit des Untergrunds, beispielsweise die Bodenart, unmittelbar zur Steuerung von im Heckbereich der Arbeitsmaschine montierten Bodenbearbeitungsgeräten verwendet wird.

Weitere erfindungsgemäße Merkmale ergeben sich aus den Patentansprüchen, der Beschreibung der Ausführungsbeispiele und den Figuren. Die Erfindung wird im Folgenden an einem nicht ausschließlichen Ausführungsbeispiel näher erläutert.

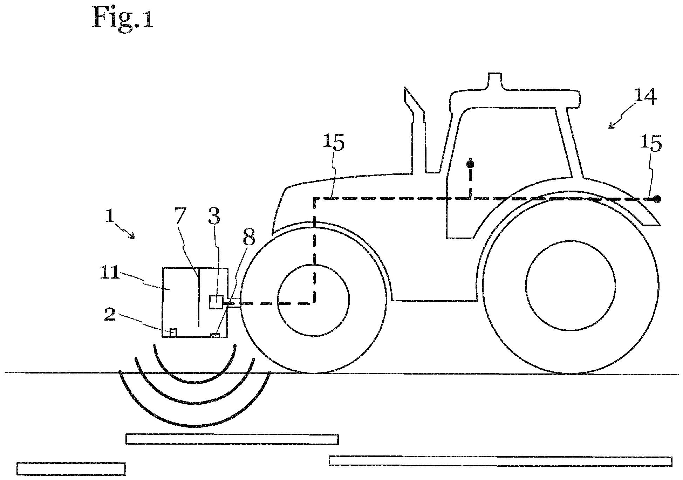

Fig. 1 zeigt schematisch einen Traktor 14 mit einem Frontgewicht 1, das eine Betonfüllung 11 aufweist und an einer Halterung im Frontbereich des Traktors 14 angeordnet ist. Bei der Halterung kann es sich insbesondere um ein an der Arbeitsmaschine vorgesehenes Fronthubwerk handeln. Im Frontgewicht 1 sind Sende- und Empfangsspulen in einem U-förmigen, nach unten offenen Sensoraufnahmebereich 2 angeordnet. Die Sende- und Empfangsspulen bestimmen die Beschaffenheit des Untergrundes und übermitteln entsprechende Daten über eine, in einem seitlich offenen Elektronikaufnahmebereich 3 angeordnete Elektronikeinheit an eine zentrale Steuereinheit im Traktor 14. Weiters sind Steuerleitungen 15 von der zentralen Steuereinheit im Traktor 14 an das Heck des Traktors vorgesehen, über die Geräte zur Bodenbearbeitung gesteuert werden können. Weiters ist im unteren Bereich des Frontgewichts 1 ein ultraschallbasierter Abstandssensor 8 vorgesehen. Um zu verhindern, dass elektromagnetische Signale der Elektronikeinheit oder des Traktors 14 die von den Sende- und Empfangsspulen gesendeten und empfangenen Messgrößen stören, ist eine metallische Abschirmplatte 7 im Frontgewicht 1 derart angeordnet, dass sie den Sensoraufnahmebereich 2 vom Elektronikaufnahmebereich 3 trennt.

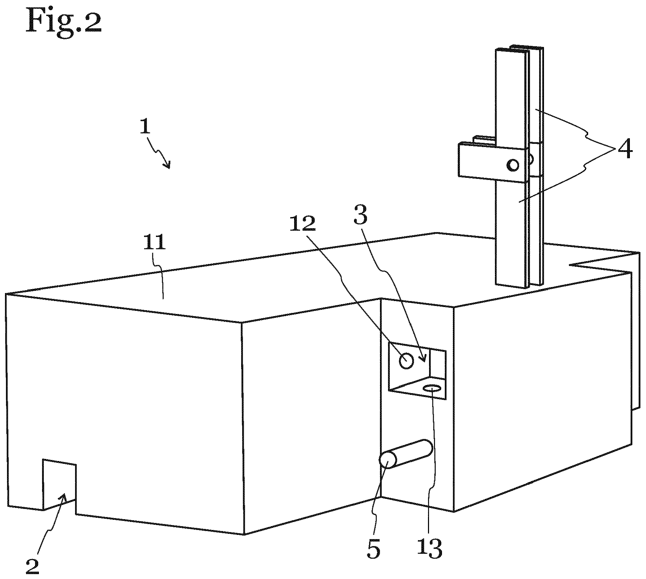

Fig. 2 zeigt eine dreidimensionale Ansicht eines Ausführungsbeispiels einer erfindungsgemäßen Vorrichtung in einem Frontgewicht 1 einer landwirtschaftlichen Arbeitsmaschine. Die Vorrichtung umfasst eine Sende- und eine Empfangsspule, wobei die Sendespule zur Erzeugung eines elektromagnetischen Primärfelds und die Empfangsspule zum Empfangen des im Untergrund durch das Primärfeld induzierten elektromagnetischen Sekundärfelds eingerichtet ist. Die Sende- und Empfangsspule ist in einem Sensoraufnahmebereich 2 in einem Frontgewicht 1 mit Betonfüllung 11 für eine landwirtschaftliche Arbeitsmaschine angeordnet. Aus Gründen der Übersichtlichkeit ist die Sende- und Empfangsspule in Fig. 2 nicht dargestellt.
   Der Sensoraufnahmebereich 2 ist im unteren Bereich des Frontgewichts 1 vorgesehen, U-förmig und nach außen offen. Räumlich getrennt davon ist ein Elektronikaufnahmebereich 3 vorgesehen, der ebenfalls nach außen offen ist. Im Elektronikaufnahmebereich 3 ist eine erste Öffnung 12 und eine zweite Öffnung 13 vorgesehen, welche zur Verbindung der Elektronikeinheiten mit den Sende- und Empfangsspulen und dem (nicht dargestellten) Distanzsensor 8 dienen. Weiters sind im Frontgewicht 1 in herkömmlicher Weise ein Träger 4 sowie Fanghaken 5 angeordnet, um das Frontgewicht 1 an einem Fronthubwerk einer landwirtschaftlichen Arbeitsmaschine, insbesondere eines Traktors, anzubringen.

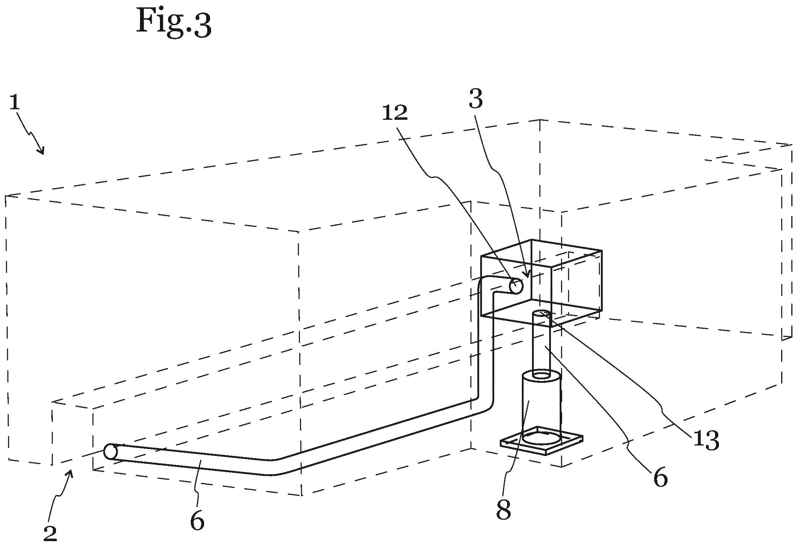

Fig. 3 zeigt eine schematische Ansicht des Frontgewichts 1, wobei die Betonfüllung 11 entfernt wurde. Im Inneren der Betonfüllung 11 finden sich Kunststoffrohre 6, welche den Sensoraufnahmebereich 2 mit dem Elektronikaufnahmebereich 3, und den Elektronikaufnahmebereich 3 mit einem Distanzsensor 8 verbinden. Der Distanzsensor 8 ist vom Sensoraufnahmebereich 2 räumlich getrennt angeordnet, um zu verhindern, dass die Messung durch den Distanzsensor 8 gestört oder beeinflusst wird. Zu diesem Zweck sind auch die Kunststoffrohre 6, wie ersichtlich, um die (nicht dargestellte) Abschirmplatte 7 herum geführt.

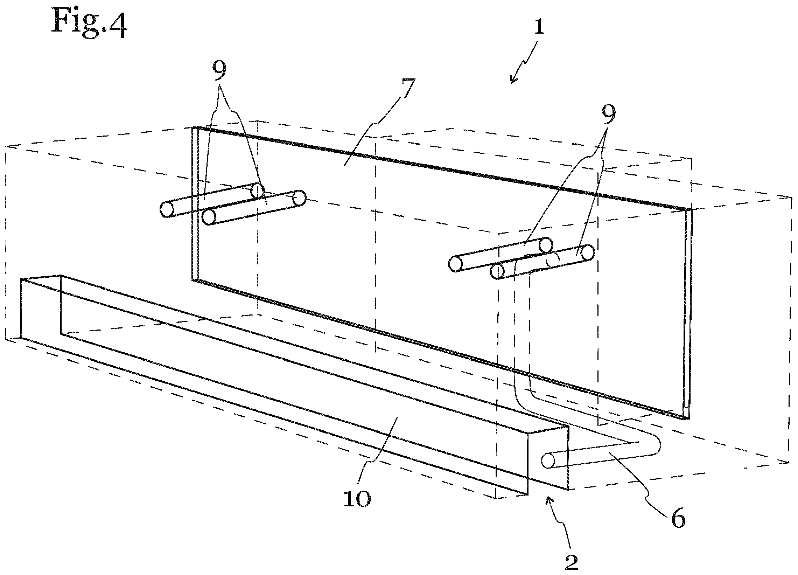

Fig. 4 zeigt eine schematische Ansicht des Frontgewichts 1 mit Darstellung der in der Betonfüllung integrierten Abschirmplatte 7 in Form einer Stahlplatte. Die Stahlplatte trennt den Sensoraufnahmebereich 2 von den anderen elektrischen und elektronischen Komponenten im Frontgewicht 1, und schützt den Sensoraufnahmebereich 2 insbesondere auch vor elektromagnetischen Einflüssen der Arbeitsmaschine selbst.

Die Kunststoffrohre 6 sind zu diesem Zweck an der Abschirmplatte 7 vorbeigeführt, um keine asymmetrische Abschirmwirkung zu verursachen. Zur Erhöhung der Stabilität des Frontgewichts 1 sind in der Betonfüllung 11 Stahlstangen 9 angeordnet. Diese befinden sich über dem Sensoraufnahmebereich 2, um die Sende- und Empfangssignale nicht zu beeinflussen. Weiters dargestellt ist in dieser Figur eine separate und vorzugsweise Sensorabschirmung 10, welche in den Sensoraufnahmebereich 2 eingeführt ist und vorzugsweise entnehmbar ist, um den Bodensensor auch ohne das Frontgewicht 1 benutzen zu können.

Die Erfindung beschränkt sich nicht auf die im konkreten Ausführungsbeispiel gezeigten Merkmale sondern umfasst sämtliche Vorrichtungen und Verfahren im Rahmen der nachfolgenden Patentansprüche.

### Bezugszeichenliste

- 1: Frontgewicht
- 2: Sensoraufnahmebereich
- 3: Elektronikaufnahmebereich
- 4: Träger
- 5: Fanghaken
- 6: Kunststoffrohre
- 7: Abschirmplatte
- 8: Abstandssensor
- 9: Stahlstangen
- 10: Sensorabschirmung
- 11: Betonfüllung
- 12: Erste Öffnung
- 13: Zweite Öffnung
- 14: Traktor
- 15: Steuerleitung

## Patentansprüche

1. Vorrichtung, insbesondere Bodensensor, zur Erfassung der Beschaffenheit eines Untergrunds, umfassend zumindest eine Sendespule und zumindest eine, vorzugsweise vier, Empfangsspulen, wobei die Sendespule zur Erzeugung eines elektromagnetischen Primärfelds und die Empfangsspule zum Empfangen des im Untergrund durch das Primärfeld induzierten elektromagnetischen Sekundärfelds eingerichtet ist, **dadurch gekennzeichnet, dass** die Sendespule und die Empfangsspule in einem Frontgewicht (1) für eine landwirtschaftliche Arbeitsmaschine angeordnet sind, und dass im Frontgewicht (1) ein Sensoraufnahmebereich (2) zur Aufnahme der Sende- und Empfangsspulen und ein davon räumlich getrennt angeordneter Elektronikaufnahmebereich (3) zur Aufnahme der Steuerelektronik vorgesehen sind, wobei Sensoraufnahmebereich (2) und Elektronikaufnahmebereich (3) durch eine vorzugsweise Stahl umfassende, elektromagnetische Strahlen abschirmende Abschirmplatte (7) getrennt sind.

2. Vorrichtung nach Anspruch 1, **dadurch gekennzeichnet, dass** ein Anschluss vorgesehen ist, um die Abschirmplatte (7) mit einer externen elektrischen Erdungsleitung zu verbinden.

3. Vorrichtung nach einem der Ansprüche 1 oder 2, **dadurch gekennzeichnet, dass** das Frontgewicht (1) einen Träger (4) zur Montage des Frontgewichts (1) an einer landwirtschaftlichen Arbeitsmaschine aufweist, wobei die Abschirmplatte (7) zwischen dem Träger (4) und dem Sensoraufnahmebereich (2) angeordnet ist.

4. Vorrichtung nach einem der Ansprüche 1 bis 3, **dadurch gekennzeichnet, dass** die Sende- und Empfangsspulen im Sensoraufnahmebereich (2) in einer vorzugsweise U-förmigen und nach unten offenen, vorzugsweise entnehmbaren Sensorabschirmung (10) angeordnet sind, wobei die Sensorabschirmung (10) vorzugsweise mit einem Anschluss versehen ist, um sie mit einer externen elektrischen Erdungsleitung zu verbinden.

5. Vorrichtung nach einem der Ansprüche 1 bis 4, **dadurch gekennzeichnet, dass** ein vorzugsweise ultraschallbasierter Abstandssensor (8) zur Bestimmung des Abstandes der Vorrichtung zum Untergrund vorgesehen ist.

6. Vorrichtung nach einem der Ansprüche 1 bis 5, **dadurch gekennzeichnet, dass** zumindest ein Neigungssensor zur Bestimmung der Neigung der Vorrichtung relativ zum Untergrund vorgesehen ist.

7. Vorrichtung nach einem der Ansprüche 1 bis 6, **dadurch gekennzeichnet, dass** zumindest ein Lokalisierungsmodul, vorzugsweise ein GPS-Modul, vorgesehen ist.

8. Vorrichtung nach einem der Ansprüche 1 bis 7, **dadurch gekennzeichnet, dass** zur Gewährleistung der mechanischen Stabilität eine Stahlstangenkonstruktion vorgesehen ist, die vorzugsweise derart im Frontgewicht (1) angeordnet ist, dass sie über dem Sensoraufnahmebereich (2) liegt.

9. Vorrichtung nach einem der Ansprüche 1 bis 8, **dadurch gekennzeichnet, dass** Mittel zum Anschluss einer externen Recheneinheit und/oder eines externen Terminals vorgesehen sind.

10. Vorrichtung nach einem der Ansprüche 1 bis 9, **dadurch gekennzeichnet, dass** der Sensoraufnahmebereich (2) und der Elektronikaufnahmebereich (3) durch Kunststoffrohre (6) miteinander verbunden sind, wobei die Kunststoffrohre neben oder unter der Abschirmplatte (7) geführt sind.

11. Landwirtschaftliche Arbeitsmaschine, insbesondere Zugmaschine, umfassend eine Vorrichtung nach einem der Ansprüche 1 bis 10.

12. Landwirtschaftliche Arbeitsmaschine nach Anspruch 11, **dadurch gekennzeichnet, dass** eine Recheneinheit zur Umrechnung der empfangenen Signale in elektrische Leitfähigkeitswerte und Bodenparameter, beispielsweise Dichte, Feuchtigkeit und Bodenart, vorgesehen ist.

13. Landwirtschaftliche Arbeitsmaschine nach einem der Ansprüche 11 oder 12, **dadurch gekennzeichnet, dass** das Frontgewicht (1) an einem Fronthubwerk der Arbeitsmaschine montiert ist.

14. Landwirtschaftliche Arbeitsmaschine nach einem der Ansprüche 11 bis 13, **dadurch gekennzeichnet, dass** an der Arbeitsmaschine Steuerausgänge zur Ansteuerung externer Bodenbearbeitungsgeräte vorgesehen sind.

15. Verfahren zum Betrieb einer landwirtschaftlichen Arbeitsmaschine nach einem der Ansprüche 11 bis 14, **dadurch gekennzeichnet, dass** während einer Arbeitsfahrt der Arbeitsmaschine die Bodenparameter des aktuell zu bearbeitenden Bodenabschnittes detektiert werden.

## Claims

1. A device, in particular a soil sensor, for detecting the condition of a ground, wherein the soil sensor comprises at least one transmitter coil and at least one, preferably four, receiver coil(s), wherein the transmitter coil is configured to generate an electromagnetic primary field and the receiver coil is configured to receive the electromagnetic secondary field induced in the ground by the primary field, **characterized in that** the transmitter coil and the receiver coil are arranged in a front weight (1) for an agricultural machine, and **in that** in the front weight (1) a sensor accommodating section (2) for accommodating the transmitter and receiver coils and a spatially separated electronics accommodating section (3) for accommodating the control electronics are provided, wherein the sensor accommodating section (2) and the electronics accommodating section (3) are separated by a screening plate (7) which preferably comprises steel and screens from electromagnetic radiation.

2. The device according to claim 1, **characterized in that** a connector is provided for connecting the screening plate (7) to an external electrical ground line.

3. The device according to one of claims 1 or 2, **characterized in that** the front weight (1) has a beam (4) for mounting the front weight (1) on an agricultural machine, wherein the screening plate (7) is arranged between the beam (4) and the sensor accommodating section (2).

4. The device according to one of claims 1 to 3, **characterized in that** the transmitter and receiver coils are arranged in the sensor accommodating section (2) in a preferably U-shaped and downwardly open, preferably removable sensor screen (10), wherein the sensor screen (10) is preferably provided with a connector for connecting it to an external electrical ground line.

5. The device according to one of claims 1 to 4, **characterized in that** a preferably ultrasound based distance sensor (8) is provided to determine the distance of the device to the ground.

6. The device according to one of claims 1 to 5, **characterized in that** at least one inclination sensor is provided to determine the inclination of the device relative to the ground.

7. The device according to one of claims 1 to 6, **characterized in that** at least one localisation module, preferably a GPS module, is provided.

8. The device according to one of claims 1 to 7, **characterized in that** for guaranteeing the mechanical stability a steel bar structure is provided that is preferably arranged in the front weight (1) in such a way that it is arranged above the sensor accommodating section (2).

9. The device according to one of claims 1 to 8, **characterized in that** means for connecting an external computing unit and/or an external terminal are provided.

10. The device according to one of claims 1 to 9, **characterized in that** the sensor accommodating section (2) and the electronics accommodating section (3) are connected to each other by plastic pipes (6), wherein the plastic pipes are guided next to or beneath the screening plate (7).

11. An agricultural machine, in particular a tractor, comprising a device according to one of claims 1 to 10.

12. The agricultural machine according to claim 11, **characterized in that** a computing unit is provided for converting the received signals into electrical conductivity values and soil parameters, for example density, moisture and soil type.

13. The agricultural machine according to one of claims 11 or 12, **characterized in that** the front weight (1) is mounted on a front lifting gear of the machine.

14. The agricultural machine according to one of claims 11 to 13, **characterized in that** control outputs for controlling external soil cultivation devices are provided on the machine.

15. A method for operating an agricultural machine according to one of claims 11 to 14, **characterized in that** the soil parameters of the soil section currently to be worked are detected during a work run of the machine.

## Revendications

1. Dispositif, en particulier un capteur de sol, pour détecter la nature d'un terrain, comprenant au moins une bobine émettrice et au moins une, de préférence quatre, bobine(s) réceptrice(s), dans lequel la bobine émettrice est agencée pour produire un champ primaire électromagnétique et la bobine réceptrice est agencée pour recevoir le champ secondaire électromagnétique induit dans le terrain par le champ primaire, **caractérisé en ce que** la bobine émettrice et la bobine réceptrice sont disposées dans un poids avant (1) d'une machine agricole, et **en ce qu'**il est prévu dans le poids avant (1) une zone de réception de capteur (2) pour recevoir les bobines d'émission et de réception et une zone de réception d'électronique (3) spatialement discrète pour recevoir l'électronique de commande, la zone de réception de capteur (2) et la zone de réception d'électronique (3) étant séparées par une plaque de blindage (7) qui est constituée de préférence d'acier et qui protège contre des rayons électromagnétiques.

2. Dispositif selon la revendication 1, **caractérisé en ce qu'**une borne est prévue pour raccorder la plaque de blindage (7) à une ligne électrique de mise à la terre externe.

3. Dispositif selon l'une des revendications 1 ou 2, **caractérisé en ce que** le poids avant (1) comprend un support (4) pour le montage du poids avant (1) sur une machine agricole, la plaque de blindage (7) étant disposée entre le support (4) et la zone de réception de capteur (2).

4. Dispositif selon l'une des revendications 1 à 3, **caractérisé en ce que** les bobines émettrices et réceptrices sont disposées dans la zone de réception de capteur (2) dans un blindage de capteur (10) de préférence en forme de U et ouvert vers le bas, de préférence amovible, le blindage de capteur (10) étant de préférence pourvu d'une borne pour le raccordement à une ligne électrique de mise à la terre externe.

5. Dispositif selon l'une des revendications 1 à 4, **caractérisé en ce qu'**un capteur de distance (8), de préférence à ultrasons, est prévu pour déterminer la distance du dispositif par rapport au sol.

6. Dispositif selon l'une des revendications 1 à 5, **caractérisé en ce qu'**au moins un capteur d'inclinaison est prévu pour déterminer l'inclinaison du dispositif par rapport au sol.

7. Dispositif selon l'une des revendications 1 à 6, **caractérisé en ce qu'**il est prévu au moins un module de localisation, de préférence un module GPS.

8. Dispositif selon l'une des revendications 1 à 7, **caractérisé en ce que**, pour assurer la stabilité mécanique, il est prévu une construction en barres d'acier, qui est disposée de préférence dans le poids avant (1) de telle sorte qu'elle se trouve au-dessus de la zone de réception de capteur (2).

9. Dispositif selon l'une des revendications 1 à 8, **caractérisé en ce que** des moyens pour connecter une unité de calcul externe et/ou un terminal externe sont prévus.

10. Dispositif selon l'une des revendications 1 à 9, **caractérisé en ce que** la zone de réception de capteur (2) et la zone de réception d'électronique (3) sont reliées entre elles par des tubes en plastique (6), les tubes en plastique étant guidés à côté de ou sous la plaque de blindage (7).

11. Machine agricole, en particulier un tracteur, comprenant un dispositif selon l'une des revendications 1 à 10.

12. Machine agricole selon la revendication 11, **caractérisée en ce qu'**une unité de calcul est prévue pour convertir les signaux reçus en valeurs de conductivité électrique et en paramètres du sol, par exemple la densité, l'humidité et le type de sol.

13. Machine agricole selon l'une des revendications 11 ou 12, **caractérisée en ce que** le poids avant (1) est monté sur un dispositif de levage avant de la machine.

14. Machine agricole selon l'une des revendications 11 à 13, **caractérisée en ce que** des sorties de commande pour la commande de dispositifs externes de culture du sol sont prévues sur la machine.

15. Procédé pour faire fonctionner une machine agricole selon l'une des revendications 11 à 14, **caractérisé en ce que**, lors d'un déplacement de travail de la machine, les paramètres du sol de la section de sol à cultiver actuellement sont détectés.
